# EUROPEAN PATENT APPLICATION

(11) **EP 4 275 586 A1**
(43) Date of publication of application: **15.11.2023**
(21) Application number: 21917320.0
(22) Date of filing: 29.12.2021
(51) Int. Cl.: A61B 1/06, A61B 1/07

(54) **ILLUMINATION DEVICE FOR ENDOSCOPE**

(30) Priority: 07.01.2021 CN 202120035628 U
(71) Applicant: Chen, Yongrui, Shanghai 201108 (CN)
(72) Inventor: KUANG, Jin, Shanghai 201108 (CN)
(74) Representative: Danubia Patent & Law Office LLC
(86) International application number: PCT/CN2021/142305
(87) International publication number: WO 2022/148281

(57) **Abstract**

The invention relates to an illumination device for use in an endoscope, which comprises a plastic light guide component, an astigmatic lens which is coaxial with the plastic light guide component and arranged at the end of the plastic light guide component, and a head base. A first channel is arranged inside the head base and the plastic light guide component penetrates and is fixed in the first channel. The astigmatic lens is installed in the front end of the head base. Compared with the prior art, the invention has the advantages of simpler assembly process, better light divergence effect, simple processing process, low cost and higher yield rate.

## Description

### BACKGROUND OF THE INVENTION

### 1. Technical Field

The invention relates to the technical field of endoscopic instruments, in particular to an illumination device for use in an endoscope.

### 2. Description of the Related Art

Endoscopic technology has been widely used in the medical industry. Doctors can observe tissue morphologies and pathological conditions of internal organs directly and efficiently with endoscopes, and then get targeted treatment plans, and even can perform minimally invasive surgery directly through the endoscope. All electronic endoscopes shall be equipped with a light source device. The light source device emits light, the light is incident on the illumination lens at the front end of the endoscope part via an optical fiber, and the observation area is illuminated via the illumination lens.

The illumination device for use in an endoscope in the prior art use a lens group composed of two or three lenses to complete the light divergence. The illumination device of the prior art is complicated to assemble and costly. Therefore, it is necessary to provide an endoscopic illumination device with simpler assembly process and lower cost.

### SUMMARY OF THE INVENTION

The invention relates to an illumination device for use in an endoscope, which comprises a plastic light guide component, an astigmatic lens which is coaxial with the plastic light guide component and arranged at the end of the plastic light guide component, and a head base. A first channel is arranged inside the head base and the plastic light guide component penetrates and is fixed in the first channel. The astigmatic lens is installed in the front end of the head base.

In some embodiments, the astigmatic lens is a convex lens with an astigmatic angle of 120 degrees.

In some embodiments, the upper portion of the astigmatic lens is a convex surface facing the object side, and the lower portion of the astigmatic lens is a cylinder facing the plastic light guide component.

In some embodiments, the astigmatic lens has a diameter of 0.9mm, the convex surface of the upper portion of the astigmatic lens has a circular arc shape with a radius of 0.55mm, the height of the cylinder of the lower portion of the astigmatic lens is 0.5mm + 0.03mm.

In some embodiments, the astigmatic lens has a diameter of 0.6mm, the convex surface of the upper portion of the astigmatic lens has a circular arc shape with a radius of 0.55mm, the height of the cylinder of the lower portion of the astigmatic lens is 0.5mm + 0.03mm.

In some embodiments, the diameter of the astigmatic lens is larger than the diameter of the first channel.

In some embodiments, the end of the first channel of the head base is provided with a position limit step, the position limit step has the same diameter as the astigmatic lens, and the astigmatic lens can be embedded in the step.

In some embodiments, the depth of the position limit step is smaller than the height of the astigmatic lens.

In some embodiments, a second channel is further provided at the lower portion of the head base.

Compared with the prior art, the invention has the advantages of simpler assembly process, better light divergence effect, simple processing process, low cost and higher yield rate.

### BRIEF DESCRIPTION OF THE DRAWINGS

The present invention will become more fully understood from the detailed description given herein and the accompanying drawings which are given by way of illustration only, and thus are not limitative of the present invention, and wherein:
FIG. 1 is a structural schematic diagram of an embodiment of the invention;
FIG. 2 is a structural schematic diagram of the head base of the invention;
FIG. 3 is an exemplary view of the use of the astigmatic lens and the light guide component of the invention;
FIG. 4 is the structural diagram of the first embodiment of the astigmatic lens of the invention;
FIG. 5 is the structural diagram of the second embodiment of the astigmatic lens of the invention.

### DETAILED DESCRIPTION OF EMBODIMENTS OF THE INVENTION

For the purpose of more clearly understanding the technical contents of the invention, the following embodiment examples are cited for detailed explanation.

Descriptions of orientation and structural location such as upper, lower, upper portion, lower portion, upper part, lower part, etc. in this description are only for the convenience of explanation of the invention and shall not be interpreted as restrictive interpretation.

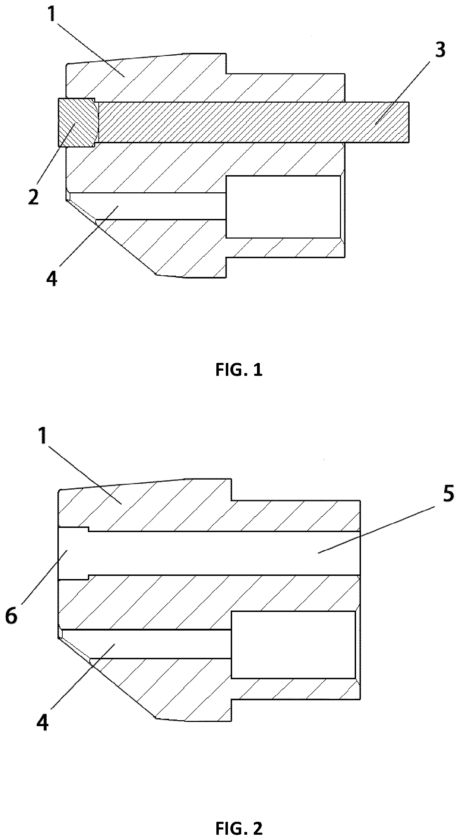

FIG. 1 is the structural diagram of the first embodiment of the invention. The embodiment includes a plastic light guide component 3, an astigmatic lens 2 which is coaxial with the plastic light guide component 3 and arranged at the end of the plastic light guide component 3, and a head base 1. A first channel 5 is arranged inside the head base 1 and the plastic light guide component 3 penetrates and is fixed in the first channel 5. The astigmatic lens 3 is installed in the front end of the head base 1. The astigmatic lens 3 is a convex lens with a plane surface on one side and a convex surface on the other side, the convex surface facing the plastic light guide component 3, the diameter of which is greater than the diameter of the first channel 5. The end of the first channel 5 is provided with a position limit step 6 which has the same diameter as the astigmatic lens 3. The astigmatic lens 3 can be embedded into the position limit step 6, and the depth of the position limit step 6 is smaller than the thickness of the astigmatic lens 3. When the astigmatic lens 3 is embedded into the position limit step 6, the upper part of the astigmatic lens 3 is exposed. The lower part of the head base is further provided with a second channel 4.

FIG. 2 shows an embodiment of the head base of the invention. In this embodiment, the head base is provided with a first channel 5 and a second channel 4. A light guide component can be inserted into the first channel 5. A position limit step 6 is provided at an end of the first channel, and a convex lens is embedded into the position limit step. The second channel 4 is disposed below the first channel 5, a camera device can be installed in this second channel 4 as an observation channel.

In a preferred embodiment, the head base of the invention can be provided with a third channel, which is used as a channel for surgical instruments.

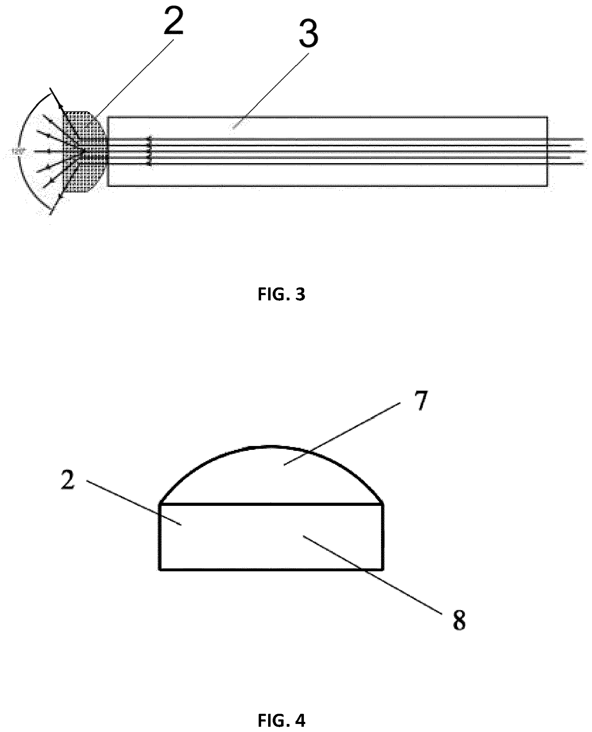

As shown in FIG.3, it is an exemplary diagram of the astigmatic lens and the light guide component of the invention in use. Parallel light is directed into astigmatic lens 2 through the plastic light guide component 3, which diverges the light into a uniform light with a divergence angle of 120 degrees to achieve the illumination function of the endoscope during use.

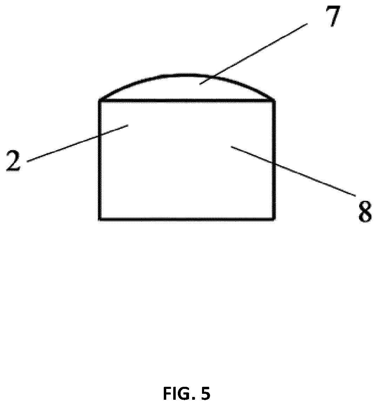

FIG. 4 and FIG. 5 are structure diagrams of the first and second embodiments of the astigmatic lens of the invention, respectively. The astigmatic lens 2 in the embodiments are all convex lens, and the upper part of the astigmatic lens is a convex surface facing the object side, and the lower part is a cylinder facing the plastic light guide component. Preferably, the astigmatic lens of the invention can be spherical convex lens.

In the first embodiment of the astigmatic lens 2 as shown in FIG. 4. The upper portion of the astigmatic lens 7 is circular-arc and the lower portion of the astigmatic lens 8 is cylindrical. The diameter of the astigmatic lens 2 is 0.9 mm, and the arc radius of the upper portion of the astigmatic lens 7 is 0.55 mm, The lower portion of the astigmatic lens 8 is cylindrical and has a height of 0.5mm + 0.03mm.

The parameters of the astigmatic lens 2 of the embodiment are:

| Material | H-ZLAF4LA |
|---|---|
| Radius of convexity | 0.55mm |
| Radius of curvature | Infinite |
| Effective radius | 0.75mm |
| Eccentricity | 1.0' |
| Newton Ring | 5 |

In the second embodiment of the astigmatic lens 2 as shown in FIG. 5. The upper portion of the astigmatic lens 7 is circular-arc and the lower portion of the astigmatic lens 8 is cylindrical. The diameter of the astigmatic lens 2 is 0.6 mm, and the arc radius of the upper portion of the astigmatic lens 7 is 0.55 mm, The lower portion of the astigmatic lens 8 is cylindrical and has a height of 0.5*mm* ± 0.03*mm*.

The parameters of the astigmatic lens 2 of the embodiment are:

| Material | H-ZLAF4LA |
|---|---|
| Radius of convexity | 0.55mm |
| Radius of curvature | Infinite |
| Effective radius | 0.5mm |
| Eccentricity | 1.0' |
| Newton Ring | 3 |

Immersion sterilization at normal temperature is one of the sterilization methods for electronic endoscope, but there is still a certain probability of infection, and the disinfectant liquid can easily penetrate into the gaps inside the endoscope to form liquid effusions, which will affect the light path. Special-purpose endoscopes, such as rigid endoscopes used in minimally invasive surgery, require higher levels of sterilization, which are sterilized at high temperatures and pressures to further reduce the probability of infection. Because the thermal expansion coefficients of different materials are different, the element with complex structure and many parts is easy to be deformed or shifted, which will directly affect illuminating, videoing and imaging of the body cavity to be observed.

In order to overcome such defects, the invention optimizes the composition of the illumination structure of the prior art, which is mainly reflected in the aspects of reduction of components and simplification of the structure, eliminates the troubles during repeated sterilization of the endoscope, and extends the service life of the endoscope. Specifically, the astigmatic lens group originally composed of two or three lenses is reduced to one astigmatic lens without affecting specific functions and effects, and the astigmatic lens is directly fixed in the head base, thereby eliminating the lens frame or sleeve. Although the design is simple in structure, the astigmatic lens can be fixed at the specified fitting position without deviation, the hidden trouble of liquid accumulation is eliminated, the optical path is stable, and the installation is convenient. Compared with the prior art, the invention has the advantages of simpler assembly process, better light divergence effect, simple processing process, low cost and higher yield rate.

In this specification, the invention has been described with reference to specific embodiments thereof. However, it is evident that various modifications and variations may be made without departing from the spirit and scope of the invention. Accordingly, the specification and the drawings are to be considered as illustrative rather than restrictive.

### LIST OF REFERENCE SIGNS

- 1: head base
- 2: Astigmatic lens
- 3: Plastic light guide component
- 4: Second channel
- 5: First channel
- 6: Position limit step
- 7: Upper portion of astigmatic lens
- 8: Lower portion of astigmatic lens.

## Claims

1. An illumination device for use in an endoscope, comprising a plastic light guide component, an astigmatic lens which is coaxial with the plastic light guide component and arranged at the end of the plastic light guide component, and a head base;
a first channel is arranged inside the head base and the plastic light guide component penetrates and is fixed in the first channel;
the astigmatic lens is installed in the front end of the head base.

2. The illumination device according to claim 1, wherein the astigmatic lens is a convex lens.

3. The illumination device according to claim 2, wherein the upper portion of the astigmatic lens is a convex surface facing the object side, and the lower portion of the astigmatic lens is a cylinder facing the plastic light guide component.

4. The illumination device according to claim 3, wherein the astigmatic lens has a diameter of 0.9 mm, the convex surface of the upper portion of the astigmatic lens has a circular arc shape with a radius of 0.55 mm, the height of the cylinder of the lower portion of the astigmatic lens is 0.5mm + 0.03mm.

5. The illumination device according to claim 3, wherein the astigmatic lens has a diameter of 0.6 mm, the convex surface of the upper portion of the astigmatic lens has a circular arc shape with a radius of 0.55 mm, the height of the cylinder of the lower portion of the astigmatic lens is 0.5*mm* + 0.03*mm*.

6. The illumination device according to claim 1, wherein the diameter of the astigmatic lens is larger than the diameter of the first channel.

7. The illumination device according to claim 1, wherein the end of the first channel of the head base is provided with a position limit step, the position limit step has the same diameter as the astigmatic lens, and the astigmatic lens can be embedded in the step.

8. The illumination device according to claim 7, wherein the depth of the position limit step is smaller than the height of the astigmatic lens.

9. The illumination device according to claim 1, wherein a second channel is further provided at the lower portion of the head base.
